# EUROPEAN PATENT SPECIFICATION

(11) **EP 0 627 489 B1**
(45) Date of publication and mention of the grant of the patent: **02.09.1998**
(21) Application number: 94109172.0
(22) Date of filing: 14.03.1991
(51) Int. Cl.: C12N 15/55, C12N 9/16, A61K 48/00, C12N 15/85

(54) **Methods and compositions for the treatment of malignancies in which a protein kinase is associated**
Methode und Zusammensetzungen zur Behandlung von bösartigen Entartungen, die in Zusammenhang mit einer Proteinkinase stehen
Procédés et compositions de traitements de tumeurs malignes auquelles une kinase protéique est associée

(30) Priority: 14.03.1990 US 494036
(43) Date of publication of application: 07.12.1994
(62) Divisional of application: 91906924.5
(73) Proprietor: WASHINGTON RESEARCH FOUNDATION, Seattle, WA 98105 (US)
(72) Inventor: Fischer, Edmond H. Dr., Seattle, WA 98105 (US); Krebs, Edwin G. Dr,, Seattle, WA 98112 (US); Cool, Deborah Dr., Seattle, WA 98199 (US); Tonks, Nick Dr., Cold Spring Harbor NY 11724 (US)
(74) Representative: Gowshall, Jonathan Vallance

(56) References cited:
- EP-A- 0 245 979
- CELL SIGNALLING vol. 2, no. 3 , 1990 pages 299 - 304 SOUTHEY M C;GONEZ L J;SANDRIN M S;KEMP B E; 'Active tyrosine phosphatase in immunoprecipitates of multiple isoforms of Ly-5'
- PROCEEDINGS OF THE NATIONAL ACADEMY OF SCIENCES OF USA. vol. 86 , July 1989 , WASHINGTON US pages 5257 - 5261 COOL, D.E. ET AL.; 'cDNA isolated from a human T-cell library encodes a member of the protein-tyrosine-phosphatase family'
- BIOCHEMISTRY. vol. 27, no. 24 , 29 November 1988 , EASTON, PA US pages 8695 - 8701 TONKS, N.K. ET AL.; 'Demonstration that the leukocyte common antigen CD45 is a protein tyrosine phsphatase'
- PROCEEDINGS OF THE NATIONAL ACADEMY OF SCIENCES OF USA. vol. 83 , May 1986 , WASHINGTON US pages 3194 - 3198 YU, S.F. ET AL.; 'Self-inactivating retroviral vectors of whole genes into mammalian cells'
- PROCEEDINGS OF THE NATIONAL ACADEMY OF SCIENCES OF USA. vol. 85 , October 1988 , WASHINGTON US pages 7182 - 7186 CHARBONNEAU, H. ET AL.; 'The leukocyte common antigen (CD45) : a putative receptor-linked protein tyrosine phsphatase'

## Description

### Technical Field

The present invention is generally directed toward compositions for use within methods for the treatment of malignancies in which a protein tyrosine kinase is associated. This invention is more particularly related to cancer therapies using DNA sequences encoding protein tyrosine phosphatase or mutant forms of the enzyme.

### Background of the Invention

Despite enormous investments of financial and human resources, cancer remains one of the major causes of death. A common characteristic of malignancies is uncontrolled cell growth. Cancer cells appear to have undergone a process of transformation from the normal phenotype to a malignant phenotype capable of autonomous growth. Mutation of somatic cell genes is considered to be a common primary event that results in the transformation of normal cells to malignant cells. The malignant phenotypic characteristics encoded by the mutated genes are passed on during cell division to the progeny of the transformed cells. Various genes involved with transformation have been designated as oncogenes. Oncogenes were originally identified as components of the genetic material of oncogenic viruses. The homologous genes on human chromosomes are commonly termed oncogenes or proto-oncogenes.

Numerous oncogenic viruses appear to operate by encoding a protein tyrosine kinase. This enzyme catalyzes the phosphorylation of tyrosyl residues in proteins. Changes in the state of phosphorylation of tyrosyl residues in proteins have been suggested to be involved in oncogenic transformation. The identity of the protein substrates and the mechanism by which their phosphorylation mediates phenotypic responses remain to be elucidated.

Approaches to the development of cancer therapies have, in general, centered on the use of characteristic differences between normal and malignant cells. More specifically, comparisons of normal and cancer cells have focused on transformation-dependent changes in the molecules residing at the surface of cell membranes. These molecules include glycolipids, proteins and glycoproteins. Certain molecules have been found to disappear or at least decrease greatly upon oncogenic transformation, while other molecules increase. Despite advances in this area, treatment of cancer cells by targeting tumor-associated cell surface molecules has met with limited success.

Due to the difficulties in the current approaches to cancer therapy, there is a need in the art for improved methods and compositions. The present invention fills this need, and further provides other related advantages.

### Summary of the Invention

Briefly stated, the present invention provides compositions whose uses include within methods for treating a malignancy in a warm-blooded animal, wherein a protein tyrosine kinase is associated with the malignancy. In one aspect, the composition comprises a gene transfer vehicle capable of infecting malignant cells, wherein the gene transfer vehicle carries a DNA construct comprising a DNA molecule encoding a receptor-linked protein tyrosine phosphatase. In one embodiment, the DNA molecule encodes a receptor-linked protein tyrosine phosphatase having a truncated carboxyl terminus. Preferred gene transfer vehicles for both aspects include a recombinant retrovirus or a recombinant vaccinia virus.

These and other aspects of the present invention will become evident upon reference to the following detailed description and attached drawings.

### Brief Description of the Drawings

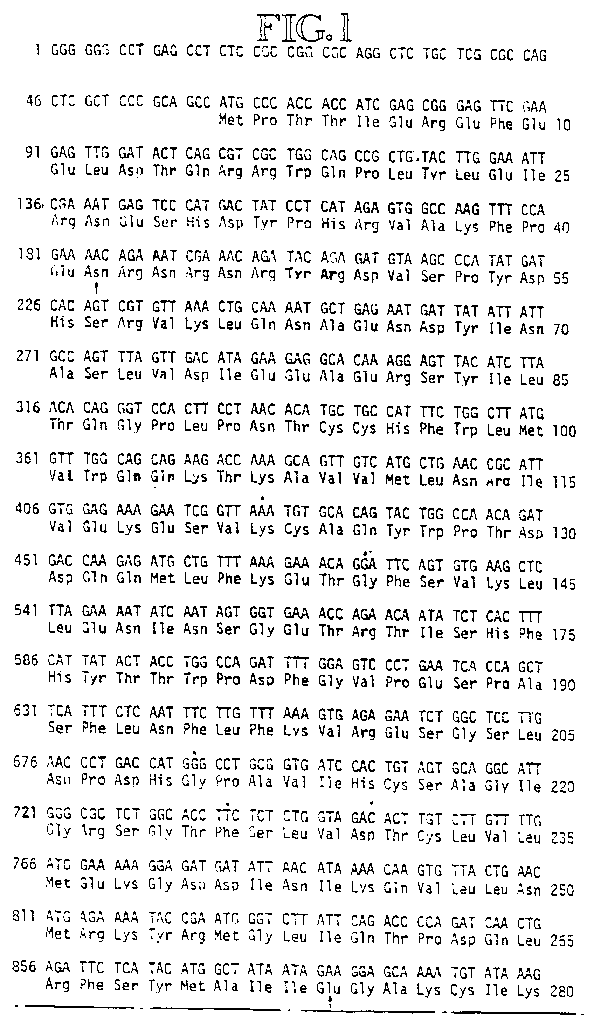

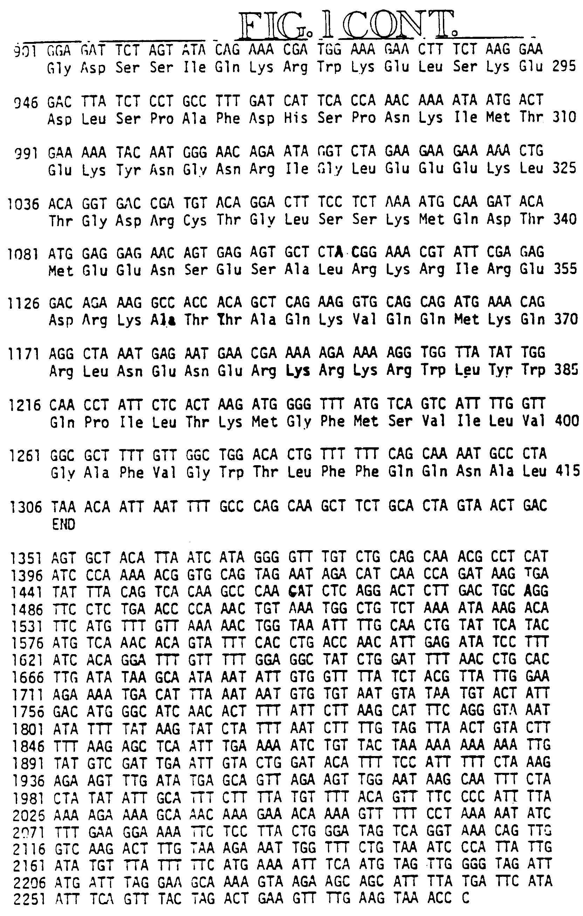

Figure 1 depicts the sequencing strategy, nucleotide sequence, and deduced amino acid sequence of human T cell cDNA encoding protein tyrosine phosphatase (PTPase). The predicted amino acid sequence of the open reading frame is shown below the nucleotide sequence. The oligonucleotide sequences used for screening the library are indicated by dots [e.g., between nucleotides 425 and 479 (probe 1), and 689 and 737 (probe 2)]. The TAA stop codon is located at nucleotide 1306 followed by a 3' untranslated end containing two possible polyadenylylation sites AATAAA at 1521 and 1677. The vertical arrows demarcate a 236-residue core segment. The schematic diagram below the nucleotide sequence depicts the sequencing strategy used. Open bar, open reading frame; solid bar, 3' untranslated end. Horizontal arrows indicate the length of sequence obtained from different sequencing oligonucleotide primers. E, EcoRI; H, HindIII, S, Sst I; X, Xba I. The scale at the bottom represents 200 nucleotides (in kbp).

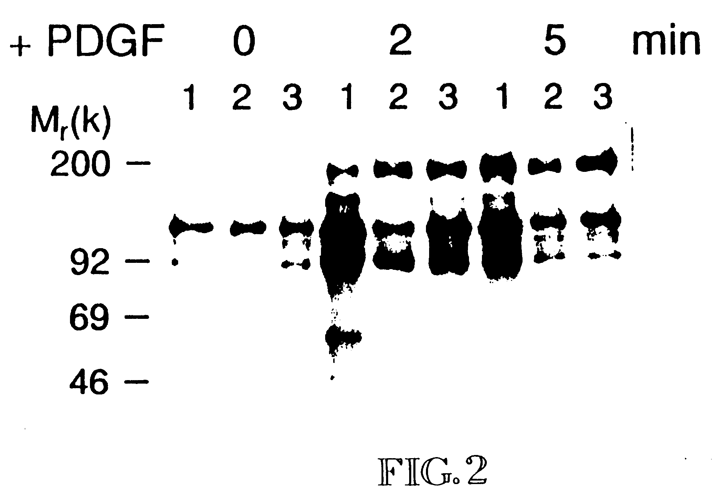

Figure 2 pictorially depicts a Western blot of phosphotyrosine-immunoprecipitated proteins following PDGF stimulation. Phosphotyrosine-containing proteins and PDGF-stimulated cells were immunoprecipitated with an anti-phosphotyrosine antibody. A SDS/7.5% Laemmli gel was used to separate the precipitated proteins followed by Western blot analysis with an anti-phosphotyrosine antibody as a probe in the blot. Detection of binding was with ¹²⁵I-labeled protein A followed by autoradiography at room temperature for 5 days. Lanes: 1, control cells; 2, cells expressing the full-length 48-kDa T-cell PTPase; 3, cells expressing the truncated form. Times of PDGF stimulation following 48 hr of serum-deprivation are indicated (0, 2, or 5 min). Standard molecular mass markers in kDa (200, myosin; 92, phosphorylase B; 69, bovine serum albumin; and 46, ovalbumin) are also shown.

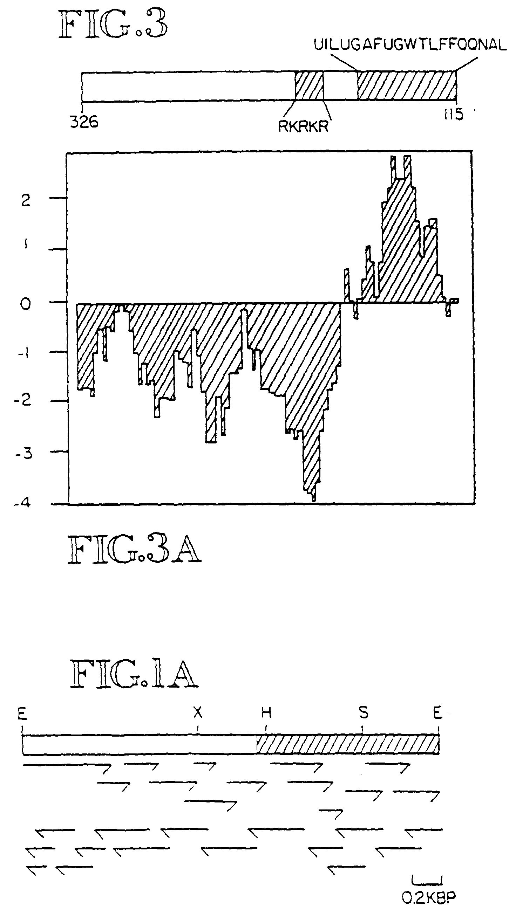

Figure 3 graphically illustrates a hydrophobicity plot of the carboxyl-terminal region in the T-cell PTPase. The hydrophobicity calculations of amino acid residues 326-415 in the carboxyl-terminal segment of the T-cell PTPase were determined as described by Kyte and Doolittle (J. Mol. Biol. 157:105-132, 1982). A computer-generated plot was obtained by using a default window of 7. The numbers above and below the 0 line represent hydrophobic and hydrophilic amino acids, respectively; the units in the *y* axis are defined as in Kyte and Doolittle. The bar above the plot depicts a putative Arg-Lys-Arg-Lys-Arg nuclear recognition signal (Van Etten et al., Cell 58:669-678, 1989) between residues 377 and 381 (solid) and a hydrophobic terminal region (396-415) (solid; 19 residues shown in single-letter code) representing the carboxyl-terminus of the molecule.

### Detailed Description of the Invention

As noted above, the present invention is directed towards compositions whose uses include within methods for treating a malignancy in which a protein tyrosine kinase is associated. The disclosure of the present invention shows the reversion of malignant transformation by transfection with human DNA encoding a protein tyrosine phosphatase.

Within one aspect of the present invention, a malignancy in which a protein tyrosine kinase is associated may be treated with a gene transfer vehicle capable of infecting malignant cells, wherein the gene transfer vehicle carries a DNA construct comprising a DNA sequence encoding a protein tyrosine phosphatase or mutant forms of the enzyme. This therapy is applicable to a wide variety of malignancies found in warm-blooded animals such as humans. Representative malignancies include breast cancer and leukemia. Within the present invention it is not necessary for all malignancies to involve tyrosine phosphorylation of the same type of protein substrate, provided that dephosphorylation of tyrosyl residues by a protein tyrosine phosphatase (whose DNA sequence has been incorporated into malignant cells) results in the reversion of the malignant phenotype.

A variety of DNA sequences encoding a protein tyrosine phosphatase ("PTPase") are suitable within the present invention. The DNA sequence encodes a receptor-linked PTPase. This type of PTPase has additional amino acid sequences that form a domain bearing traits of a cell-surface receptor. Non-receptor-linked PTPases typically have a molecular weight less than approximately 50,000. Receptor-linked PTPases typically have a higher molecular weight, e.g., 150,000-250,000, and display a wide variety of external domains.

In one embodiment of a DNA sequence useful within the present invention, the sequence encodes a wild type (i.e., a naturally occurring form) PTPase. A representative example of wild type, non-receptor-linked PTPase DNA is the sequence shown in Figure 1 for human T cell PTPase. Other examples include a DNA sequence encoding a PTPase of human placenta. A representative example of wild type, receptor-linked PTPase DNA is the DNA sequence encoding CD45 in human leukocytes.

In another embodiment, the DNA sequence used within the present invention encodes a PTPase having a truncated carboxyl terminus, i.e., missing amino acid residues normally found at or near the carboxyl terminus in the sequence of a wild type PTPase. The disclosure of the present invention provides a representative example of a DNA sequence encoding a truncated PTPase. This DNA was prepared by altering the nucleotide sequence of the cDNA encoding a T cell PTPase (Figure 1) using standard molecular biology techniques. Briefly, a deletion of a few nucleotides by site directed mutagenesis was introduced into wild type cDNA. This sequence alteration resulted in the placement of a premature translation stop signal in the open reading frame of the cDNA. The modified cDNA encodes a PTPase (molecular mass of about 37,000) with a truncated carboxyl terminus relative to the 48,000 molecular mass protein normally produced in T cells. This truncated PTPase has the amino acid sequence of Figure 1 from methionine, amino acid 1, to arginine, amino acid 317. The enzymatic activity exhibited by transfected cells expressing this truncated PTPase is higher than the activity in control cells.

It will be evident to those skilled in the art that a variety of DNA sequences encoding PTPases having truncated carboxyl termini may be employed within the present invention. In general, any number of amino acid residues may be deleted from the carboxyl terminus, provided that the resultant protein does not exhibit a significant reduction in PTPase activity. The enzymatic activity of truncated PTPases may be measured, for example, using an assay based on the measurement of a reporter group released from a labeled substrate. A suitable assay includes the one described below for the measurement of ³²P released from reduced, carboxyamidomethylated maleylated-lysozyme. As denoted in Figure 1, there is a 236-amino acid residue core identified by T cell PTPase amino acid 42 (asparagine) to amino acid 274 (glutamic acid). T cell PTPase and placenta PTPase 1B, for example, possess within this core about 65% sequence identity and approximately 80% sequence similarity (after optimizing the alignment between the two sequences). Truncated forms of PTPase may be prepared by the deletion of amino acids starting at the carboxyl terminus and moving back towards the 236-residue core. Suitable DNA sequences include those encoding a PTPase comprising an amino acid sequence of between (including) 297 to 320 amino acid residues where the sequence contains a portion of similar length to, and having at least approximately 80% sequence similarity with, this 236-residue core. This portion of a sequence need not be of identical length and it may be necessary to shift the sequence and/or insert gaps (in it or the sequence of Figure 1) in order to optimize the alignment between the two sequences. Sequence similarity is based upon sequence identity plus conservative substitutions of amino acids. Conservative substitutions include interchanges of valine and isoleucine, leucine and isoleucine, aspartic acid and glutamic acid, and others of a similar nature. A preferred DNA sequence encodes a truncated PTPase comprising the amino acid sequence of Figure 1 from methionine, amino acid 1, to an amino acid positioned between (including) leucine, amino acid 297, and arginine, amino acid 317.

Following isolation or preparation of a suitable DNA sequence encoding a wild type or truncated PTPase, the sequence is inserted into a gene transfer vehicle. Suitable gene transfer vehicles include a recombinant retrovirus or a recombinant vaccinia virus.

Retroviruses are RNA viruses that can replicate through a DNA intermediate through the action of an RNA dependent DNA polymerase. Once in DNA form, they may be stably integrated into the host cell genome and passed down from a parent cell to its progeny. Thus, retroviruses are suitable as transfer vehicles for foreign DNA into a host cell (see Shimotohno and Temin, "Formation of Infectious Progeny Virus After Ingestion of *Herpes Simplex* Thymidine Kinase Gene into DNA of an Asian Retrovirus," Cell 26:67-77, 1981; see also, Shimotohno and Temin, "Loss of Intervening Sequences in Genomic Mouse Alpha-Globin DNA Inserted in an Infections Retrovirus Vector," Nature 299:265-268, 1982; see also; Miller and Rosman, "Improved Retroviral Vectors for Gene Transfer and Expression," BioTechniques 7:980-990, 1989).

Where the retrovirus transfer vehicle is replication competent, the resultant active virus may not be appropriate for therapeutic purposes because it is also replication competent, and represents a health hazard. However, various methods have been utilized to design a retrovirus transfer vehicle which can produce a retrovirus containing foreign DNA, yet which is nevertheless replication incompetent (see U.S. Patent Nos. 4,650,754 (Temin et al.), 4,861,719 (Miller), 4,405,712 (Van de Woude et al.), and 4,885,238 (Reddel et al.), which are incorporated herein by reference; see also Jacob etal., European Patent Application Publ. No. 0178220, European Patent Application Publ. No. 0243204, PCT International Publ. No. WO 85/05629, PCT International Publ. No. WO 87/03451, PCT International Publ. No. 89/07150, and PCT International Publ. No. WO 89/09271). Various retroviruses may be used within the present invention including the Murine Sarcoma Virus (MSV) and the Moloney Murine Leukemia Virus (MoMLV). Furthermore, it will be evident to one of ordinary skill in the art that envelope sequences may be derived from amphotropic viruses such as virus 4070A.

Briefly, a mutant of the Moloney murine leukemia virus is constructed which contains a deletion of about 350 nucleotides in the *env* region of the retrovirus (see Shank and Linial, J. Virol. 36(2):450-456, 1980). This produces a virus (gag⁺,pol⁺,env⁻) deficient in encapsidation or packaging of viral RNA, which may, if cotransfected with an encapsidation positive retroviral vector (gag⁻,pol⁻,env⁺) containing a desired foreign gene, result in the production of retrovirus which is replication incompetent, yet which nevertheless produces the foreign gene (see Temin et al. U.S. Patent No. 4,650,764).

There remains, however, the possibility of recombination between the gag⁻,pol⁻,env⁺ vector and the gag⁺,pol⁺,env⁻ vector. Such recombination might produce an infectious retrovirus (gag⁺,pol⁺,env⁺), Therefore, it is particularly preferred to design recombinant retrovirus vectors which are safe transfer vehicles for foreign genes. Briefly, the retroviral genome, or provirus, contains two Long Terminal Repeats (LTR) which encode sequences for initiation and termination of retroviral transcription, including promoters, translation and termination signals, as well as enhancers. The LTR has 3 regions: U3, R, and U5. Within a preferred embodiment the U3 region (containing promoter and enhancer segments) of the right side LTR is deleted resulting in a retroviral vector which is self-inactivating (see Yu et al., "Self-Inactivating Retroviral Vectors Designed for Transfer of Whole Genes Into Mammalian Cells," Proc. Natl. Acad. Sci. USA 83:3194-3198, 1986; Markowitz et al., "A Safe Packaging Line for Gene Transfer: Separating Viral Genes on Two Different Plasmids," J. Virol. (62):1120-1124, 1988). This vector (containing a promoter and the foreign gene, i.e., U3, R, U5----promoter, gene, ----U3(del.), R, U5) is used to transfect helper cells. Viral transcription begins on the left side LTR beginning at R, and transcribes a sequence which does not contain either the left side or right side U3 region (i.e., R, U5, ---- promoter, gene ---- U3(del), R). Thus, the retroviral vector is self-inactivating yet, nevertheless, because it contains an internal promoter allows transcription of the foreign DNA.

Vaccinia viruses are eukaryotic DNA viruses that reproduce entirely within the cytoplasm of a host cell. The virus has been used for about 200 years in vaccines for inoculation against smallpox. The virus is considered non-oncogenic and relatively benign. The naturally occurring vaccinia genome can be modified to produce recombinant vaccinia by rearrangement of the natural genome, by the removal of DNA from the genome, and/or by the introduction of foreign DNA into the genome (e.g., U.S. Patent Nos. 4,769,330 and 4,886,782, which are incorporated herein by reference). Thus, recombinant vaccinia virus represents a relatively innocuous eukaryotic cloning vector useful within the present invention for the introduction of a DNA sequence encoding a PTPase into a eukaryote.

The following examples are offered by way of illustration and not by way of limitation.

### EXAMPLES

### EXAMPLE 1

### CONSTRUCTION OF A HUMAN T CELL PROTEIN-TYROSINE-PHOSPHATASE

All restriction and modifying enzymes and *in vitro* transcription and translation systems were purchased from Stratagene, La Jolla, California. Oligonucleotides were synthesized with an Applied Biosystems 380A DNA Synthesizer (Applied Biosystems, Foster City, Calif.). Radionucleotides were obtained from New England Nuclear (Boston, Mass.). Sequenase was obtained from United States Biochemical (Cleveland, Ohio). Solutions such as Denhardt's solution were prepared as described by Maniatis et al., Molecular Cloning: A Laboratory Manual, Cold Spring Harbor Lab., Cold Spring, N.Y., 1982.
A. Screening of a cDNA Library with Synthetic Oligonucleotides
   A set of complementary overlapping oligonucleotides were synthesized for each of the protein segments Lys-120-Asn-139 and Gly-209-Phe-225 from the human placenta PTPase 1B according to the method of L. Charbonneau et al., Proc. Natl. Acad. Sci. USA 86:5252-56, 1989. Prediction of the DNA sequence was based on the optimal codon choice for human amino acid sequence data (see R. Lathe, J. Mol. Biol 183:1-12, 1985). Oligonucleotides in set 1 (5'-AAGTGTGCACAGTACTGGCCGCAGAAGGAA-3' and 5'-GTTGGTATCCTCAAAGATCATCTCCCTTCTCTTCCTTC TGC-3') or in set 2 (5'-GGTCC TGTGGTGGTGCACTGCAGTGCTGGT-3' and 5'-AAGGTTCCAGTG CGCCAATACCAGCACTG-3') were annealed and labeled using ³²PdATP and ³²PdCTP and the Klenow fragment of DNA polymerase I, producing radioactive double-stranded DNA with a specific activity of 4 x 10⁷ cpm/pmol bp.
   A λgt10 recombinant cDNA phage library containing 500,000 phage was prepared from human peripheral T cell poly(A)⁺ mRNA according to the method of Littman et al., Cell 40:237-46, 1985, and plated at a density of 50,000 phage per plate. Duplicate nitrocellulose filter lifts were taken from each plate and hybridized with the oligonucleotide probes prepared above (2.5 x 10⁶ cpm per filter) in 20% formamide/5 x SSC (1 x SSC is 150 mM NaCl/15 mM sodium citrate)/2.5 x Denhardt's solution/1 mM sodium pyrophosphate/50 mM sodium phosphate buffer, pH 6.8, at 37°C for 18 hours. The filters were washed in 2 x SSC/0.2% SDS at 42°C and subjected to autoradiography for 3 days at -70°C with an intensifier screen.
   Although many recombinant phage hybridized to each probe, only one overlapping positive clone bound to both oligonucleotides.
B. DNA Sequence Analysis
   Approximately 10¹⁰ *E. coli* cells were infected with the recombinant phage selected above, and lysed by the alkali method (see Sambrook et al., Molecular Cloning: A Laboratory Manual, 2 ed., Cold Spring Harbor Laboratory Press, pp. 1.25-1.28, 1989). The amplified phage were purified by CsCl density centrifugation. DNA from the purified phage was extracted and digested with EcoRI restriction endonuclease. The EcoRI fragments were analyzed by agarose gel electrophoresis which resolved two vector DNA fragments (32 and 11 kbp) and a cloned cDNA fragment of 2.3 kilobase pairs (kbp) in length.
   One hundred nanograms of the 2.3 kbp EcoRI insert and 100 ng of the Bluescript cloning vector (Strategene, La Jolla, Calif.) were cleaved with EcoRI (a site within the β-galactosidase gene), ligated in a reaction mixture containing 50 mM Tris (pH 7.4), 10 mM MgC₂, 10 mM dithiotheritol and 2 mM ATP, and transformed into an *Escherichia coli* host (XL-Blue, Stratagene). The transformed cells were plated on agar plates containing 100 µg/ml ampicillin and 50 µg/ml X-gal (Bethesda Research Lab). White colonies (containing cDNA inserts) were selected and a miniprep of plasmid DNA was prepared and run on an agarose gel to ensure presence of an insert. A cell was selected which contained the insert and grown in LB (Luria-Bertani) medium (see Sambrook et al. *supra*, p.A.1) for 12 hours. Cells were lysed by the alkali method, and the closed circular plasmid DNA extracted and purified by CsCl centrifugation.
   The purified DNA was sequenced using the strategy illustrated in Figure 1 according to the chain-termination method of Sanger et al. (Proc. Natl. Acad. Sci. USA 74:5463-6467, 1977). Briefly, sequencing reactions contain a mixture of deoxy (including ³²PdATP) and dideoxy nucleotides. The DNA to be sequenced is denatured by boiling and annealed with 18-mer single stranded primers which recognize a precise sequence in the cDNA or vector sequence adjacent to the cloning site. The DNA is copied from the priming site utilizing the Sequenase enzyme provided in a sequencing kit (United States Biochemicals Co., Cleveland, Ohio). The exact ratios of deoxy and dideoxy nucleotides have been determined by the supplier and were used accordingly.
   Sequence analysis (Figure 1) shows that the T cell PTPase cDNA contains an open reading frame of 1305 nucleotides. A consensus sequence CC(AG)CCAUG(G) for eukaryotic initiation sites (described by Kozak, Nucleic Acids Res. 12:857-873, 1984) was found at nucleotides 56-64 encoding a putative initiator methionine. The open reading frame terminates with a TAA stop codon followed by 978 bp of 3' untranslated end. However, neither a polyadenylylation site nor a 3' poly(A)⁺ tail was observed. There are two possible AATAAA polyadenylylation signals (see Proudfoot and Brownlee, Nature 265:211-214, 1976) at sites 213 and 369 bp past the stop codon (nucleotides 1521-1526 and 1677-1682, respectively).
C. *In Vitro* Translation of T-Cell PTPase mRNA
   The Bluescript plasmid containing the T cell cDNA was made linear by HindIII restriction endonuclease digestion. mRNA was synthesized *in vitro* from 1 µg of plasmid DNA using the T7 polymerase promoter, which is positioned 5' to the T cell PTPase cDNA insert in the Bluescript cloning vector. Synthesis of mRNA was carried out in the presence of DNA, nucleotide triphosphates required for RNA, buffer and T7 Polymerase (all reagents were provided by Strategene). Following synthesis for 1 hour at 37°C, the DNA was degraded with DNAse l (Stratagene), and the proteins digested with Proteinase K (Stratagene) for 1 hour at 68°C. RNA was then extracted with phenol:chloroform (1:1) and ethanol precipitated. The mRNA (1 µg) was added to 20 µl of a rabbit reticulocyte translation system in the presence of ³⁵S methionine and protein synthesis was allowed to proceed for 30 minutes. The control reaction mixture contained mRNA produced from the linearized vector. The products were analyzed on a 10% SDS-polyacrylamide gel according to the method of Laemmli (Nature 227:680-685, 1970) and subjected to autoradiography for 18 hours. A protein product with an estimated Mᵣ of 48,000 was produced.
D. Northern Blot Analysis
   Total RNA was extracted according to the method of Cathala et al., DNA 2:329-335, 1983, from monkey brain, spleen, and thymus; human RNA was extracted from placenta and T cells. Poly(A)⁺ mRNA was purified by oligo(dT) column chromatography according to the method of Maniatis et al, *supra*, p. 197. Poly(A)⁺ mRNA (10 µg) from brain, spleen, thymus, and placenta and 20 µg of the total T cell mRNA were subjected to electrophoresis in a 0.8% formaldehyde agarose gel and transferred to nitrocellulose filter paper by the diffusion method described by Maniatis et al., *supr*a, p. 203. The filter containing RNA (Northern blot) was hybridized and denatured. ³²P-labeled cDNA insert purified from the T cell clone was used as a probe. The hybridization conditions were the same as those described for the screening of the library, except that the blot was washed in 0.1 x SSC/0.2% SDS at 50°C. The gel was exposed to film for 3 days at -70°C with an intensifier screen.
   Analysis of the blot revealed multiple bands of hybridization. The most abundant transcript (≈2.3 kb) was found in all the above tissues, although the level of expression in brain was quite low. Comparison of the thymus poly(A)⁺ mRNA with the T cell total mRNA showed at least a 20-fold enrichment of the transcript. The predominant message, whose precise length cannot be determined in the agarose gel, appears to represent the T cell PTPase cDNA since the expected length of this transcript is at least 2.5 kb including a 200-base poly(A)⁺ tail (see Perry, Annu. Rev. Biochem. 45:605-629, 1976).
E. Southern Blot Analysis
   Human genomic DNA was cleaved with the restriction endonucleases BamHI EcoRI, and HindIII. The fragments were separated in a 0.8% agarose gel and transferred to nitrocellulose filter paper by diffusion (see Maniatis, *supra*). The filter containing DNA (Southern blot) was hybridized to the denatured ³²P-labeled insert of the T cell cDNA and washed as described for the Northern blot analysis and subjected to autoradiography for 3 days at -70°C with an intensifier screen. Autoradiography of the blot revealed several bands of hybridization, indicating that either the gene is very large (>70 kbp with many introns) or that there are multiple genes in this family. It was then reprobed with the labeled cDNA using the same hybridization conditions as above, but washed under less stringent conditions, such as 2 x SSC/0.2% SDS and 45°C. No new bands of hybridization were detected.
F. Insertion of the Human T Cell PTPase Clone Into a Plasmid
   An EcoRI-HindIII fragment (1.328 kbp) was isolated from the human T cell cDNA PTPase clone obtained above. This fragment represents the entire coding region of the PTPase cDNA; including 60 b.p. from the 5' untranslated region and 22 b.p. from the 3' untranslated region. The single stranded ends produced by the restriction enzymes were removed with nuclease S1 digestion.
   Many plasmid vectors are known to one of ordinary skill in the art which are suitable for expressing DNA inserts, including pMFG (Pharmacia LKB Biotechnology, Inc., Piscataway, N.J.), and pNut (obtained from Richard Palmiter, University of Washington; see Palmiter et al., Cell 50:435-443, 1987). The pNut expression vector was cleaved with SmaI and run on an agarose gel. A 5.5 kbp fragment was isolated from the agarose gel by electroelution of the DNA into buffer. The DNA was then precipitated with ethanol. The fragment encodes a dihydrofolate reductase cDNA under the regulation of a simian virus 40 (SV40) promoter and a Zn²⁺ metallothioneine I promoter required for in vivo transcription of the newly inserted cDNA. The plasmid pNUT.TCPTP was generated by ligating the 1.3 kbp T cell PTPase cDNA fragment with the SmaI pNUT vector fragment.

### EXAMPLE 2

### CONSTRUCTION OF A TRUNCATED FORM OF THE T-CELL PTPASE

A mutation of the PTPase cDNA was performed according to the method of Kunkel et al., "Rapid and Efficient Site-Specific Mutagenesis Without Phenotypic Selection," Methods in Enzymology 154:367-382, 1987. Briefly, the 2.3 kbp cDNA EcoRI insert was ligated into the EcoRI site of an M13 mp18 vector. The ligated DNA was transformed into an *E. coli* host and M13 phage plaques were produced on an agarose plate. The DNA of these phage plaques is single stranded and can be used for DNA sequencing or for site directed mutagenesis. In particular, the DNA was purified (see Sanger, *supra*) and hybridized to the oligonucleotide 5'-GGG AAC AGA TAG AAG AAG-3'. The oligonucleotide was synthesized with an Applied Biosystems 380A DNA Synthesizer, and represents nucleotides 1004-1025 with a seven base deletion in the wild type cDNA resulting in the placement of a stop codon (TAG) into the translation open reading frame. The primed, single stranded M13 DNA was used as a template to generate double stranded DNA, one of which had the deletion. The newly synthesized heterodimer double stranded DNA was transformed into *E. coli* host and plated on agarose plates to allow the growth of M13 recombinant phage. M13 phage carrying the deletion were selected by *in situ* plaque filter hybridization (Wood et al., Proc. Natl. Acad. Sci. USA 82:1585-1588, 1985) with ³²P-labeled oligonucleotide, followed by washing 6 x SSC, at 48°C. A 1.6 kbp fragment was produced upon ThaI and SspI restriction enzyme digestion of the mutated M13 plasmid DNA. The fragment was isolated and inserted into the Smal site of the pNUT expression vector. All plasmid constructs were verified by DNA sequence analysis using the chain termination method of Sanger et al., *supra.*

### EXAMPLE 3

### PTPASE ASSAYS

Cell extracts or purified enzymes were assayed for tyrosine phosphatase using a method described by Tonks et al., in "Purification of the Major Protein-tyrosine-phosphatases of Human Placenta," J. Biol. Chem. (263):6722-6730, 1988, based on measurement of the release of ³²P from labeled substrate. Since phosphorylation of reduced, carboxyamidomethylated, maleylated (RCM)-lysozyme is not stoichiometric, concentrations are expressed in terms of ³²P phosphotyrosine. Briefly, a mixture of 0.02 ml of PTPase (diluted to less than 1 unit/ml in Buffer A) and 0.02 ml of Buffer A was warmed at 30°C for 5 minutes. Buffer A is composed of 25 mM imidazole HCl (pH 72), 1 mg/ml BSA, 0.1% (v/v) β-mercaptoethanol. The reaction was initiated by addition of 0.02 ml of ³²P-TyrRCM lysozyme (final concentration of 5 µM) that had also been preincubated to 30°C. The reaction was terminated after approximately 10 minutes by addition of 0.18 ml of 20% (w/v) trichloroacetic acid and 0.02 ml of 25 mg/ml BSA, added as a carrier protein. The suspension was vortexed, allowed to stand on ice for 10 minutes, and centrifuged at 12,500 x g for 3 minutes. A 0.2-ml aliquot of the supernatant was added to 1 ml of Aquasol scintillant and counted in a Beckman LS7000 scintillation counter. Dephosphorylation was linear with respect to time and enzyme concentration; up to 50% of the ³²P was released. Blank incubations were performed in which the PTPase was replaced by Buffer A, and total ³²P was determined by counting 0.02 ml of the substrate. Radioactivity in the blank was routinely less than 2% of total ³²P in the assay. Released ³²P was confirmed to be inorganic phosphate, rather than radioactive peptides, by the molybdate/isobutyl alcohol/benzene extraction procedure of Foulkes et al., FEBS Lett. 130:197-200, 1981. One unit of PTPase activity is defined as that amount which releases 1 nmol of phosphate/min.

For those assays requiring trypsin, 20 µl of cell extract was diluted 1:2 in buffer and treated with 1 µg of trypsin for 5 min. at 30°C. Trypsin digestion was stopped by adding 6 µg of lima bean trypsin inhibitor, followed immediately by 20 µl of substrate.

### EXAMPLE 4

### PREPARATION OF ANTI-PEPTIDE SERA

The peptide CNRNRYRDVSPFDHSRIK (identified by reference to the single-letter amino acid code) was synthesized on an Applied Biosystems Peptide Synthesizer (Applied Biosystems, Foster City, Calif.). The sequence was derived from an amino terminal region (residues Asn 43 to Lys 60 of placenta PTPase 1B enzyme (see Charbonneau et al., Proc. Natl. Acad. Sci. USA 86:5252-5256, 1989). The peptide contains an additional cysteinyl residue to facilitate cross-linking to rabbit serum albumin as a carrier protein. Polyclonal antibodies were produced by immunization of a rabbit using conventional techniques followed by collection of its sera. The serum was affinity purified or was loaded onto a cellulose Affi-Gel Blue/DE 52 column (mixed 1:1) and eluted in 20 mM Tris pH 8.0, 20 mM NaCl. Fractions were collected and analyzed for protein. The antibody still recognizes the T cell enzyme even though two amino acid substitutions are found in this domain (tyrosine for Phe-52 and valine for Ile-57). Specificity of the antibody for PTPase was verified by peptide competition experiments.

### EXAMPLE 5

### EXPRESSION OF WILD-TYPE AND TRUNCATED T-CELL PTPASE

A. BHK Cells
Baby Hamster Kidney (BHK) cells were routinely grown in Dulbecco's modified Eagle's medium containing 10% (vol/vol) heat-inactivated fetal calf serum. The cells were transfected with 10 µg of the pNut plasmid containing either the T cell PTPase DNA or the truncated T cell PTPase DNA using the calcium phosphate precipitation method (Wigler et al., Cell 16:772-785, 1974) and after 24 hours were switched to selection media containing 250 µM methotrexate. Stable colonies were isolated about 14 days post transfection.
Confluent stably transfected cells were treated with 80 µM ZnSO₄ for 12 hours in order to induce transcription of the PTPase in RNA through the metallothionine promoter. The plates were washed three times with phosphate-buffered saline (PBS) and scraped in order to remove the cells. The cells were pelleted by low speed centrifugation (500 xg) for 5 min. and the pellet lysed in either a low salt buffer (LSB) (25 mM Imidizole (pH 7.0), 2 mM MgCl₂, 1 mM EDTA, 1 mM EGTA, 0.1% β-mercaptoethanol, 0.002% PMSF, 0.1 mM Benzamidine, 1 µg/ml leupeptin, 250 mM sucrose), LSB-Triton X-100 buffer, (LSB and 0.5% Triton X-100) or KCl/CHAPS buffer (KCB) (same as LSB without sucrose and including 0.6M KCl, 1.0% CHAPS). The homogenates were dounced for 30 seconds with a Teflon® homogenizer, followed by centrifugation at 5,000 xg for 5 min. to yield a low-speed centrifugation pellet ("5P"). The supernatant was recentrifuged at 100,000 xg at 4°C for 30 minutes to yield a high-speed centrifugation supernate ("100S") and pellet ("100P").
PTPase assays (as described in Example 3) were carried out on the various fractions (Table 1).

**TABLE 1**

| PTPase Activity in BHK Cells | | | | | | | | |
|---|---|---|---|---|---|---|---|---|
| Fraction | Transfected plasmid | Total Units | | Units/mg | | Fold trypsin stimulation | % total units | |
| | | - | + | - | + | | - | + |
| A Low-speed pellet (5 P) | Control | 5.2 | 31.0 | 0.7 | 4.4 | 6.0 | 73 | 87 |
| | TC.PTPase | 11.3 | 320.0 | 1.6 | 31.0 | 19.0 | 84 | 97 |
| | TCΔC11.PTPase | 17.0 | 26.0 | 2.5 | 3.7 | 1.5 | 48 | 66 |
| | | | | | | | | |
| B High-speed pellet (100 P) | Control | 0.4 | 0.5 | 0.6 | 2.6 | 4.5 | 5 | 2 |
| | TC.PTPase | 1.0 | 5.6 | 1.0 | 5.6 | 5.0 | 5 | 2 |
| | TCΔC11.PTPase | 3.4 | 3.9 | 4.3 | 4.9 | 1.0 | 9 | 10 |
| | | | | | | | | |
| C High-speed supernatant (100 S) | Control | 1.6 | 4.0 | 0.4 | 1.1 | 3.0 | 22 | 11 |
| | TC.PTPase | 2.1 | 5.4 | 0.6 | 1.5 | 2.5 | 11 | 1 |
| | TCΔC11.PTPase | 12.5 | 7.5 | 3.4 | 2.1 | 0.6 | 43 | 24 |
| BHK cell were fractionated (5P, 100P, or 100S fractions) by centrifugation as described. The PTPase was determined in cells expressing either the control plasmid (control) or the cDNA of the full-length 48-kDA T-cell PTPase (TC.PTPase) or the truncated form (TCΔC11.PTPase). Total units of activity have been standardized to a constant amount of protein in each fraction. The signs "-" or "+" indicate assays without or with 1 µg of trypsin in the assay, respectively. "% total units" represents the percentage of total cellular activity found in each fraction. | | | | | | | | |

Essentially all of the endogenous and expressed PTPase activities (of BHK cells expressing a full-length 48-kDa human T cell PTPase) sedimented with the 5P pellet from which they could be released by 0.5% Triton X-100/0.6 m KCl. Triton alone was only partially effective, and salts alone at high concentration were totally ineffective. The low levels of activity and protein found in the 100P pellet was not considered further. Although the level of enzyme in the transfected cells was found at first to be no greater than in the controls, it could be increased considerably upon limited trypsinization (6- and 20-fold in the 5P fraction for the control and transfected cells, respectively). Under these conditions, the total activity in the transfected cells was 10 times greater than that in the controls.
A 0.5% Triton X-100 extract was subjected to Superose 12 fast protein liquid chromatography (FPLC) gel filtration. In both control and transfected cells, the PTPase activity emerged in a high molecular mass (>650 kDa) fraction. Western blot analysis of this material following CCl₃ COOH precipitation and SDS/PAGE revealed the presence of a 48-kDa immunoreactive protein. Similar results were obtained when the cells were extracted in 1% 3-[(3-cholamidopropyl)dimethylammonio]-1-propanesulfonate (CHAPS)/0.6 M KCl and then freed of detergent by dialysis against a low-salt buffer prior to gel filtration, suggesting that the formation of the high molecular mass complex was not due to the presence of detergents.
When the Triton-soluble extracts were treated with trypsin prior to gel filtration, the PTPase was eluted with an apparent molecular mass of approximately 35 kDa. Western blot analysis also revealed a band at approximately 33 kDa, suggesting that cleavage had occurred at the carboxyl terminus, since the antibody used recognized a sequence near the amino terminus of the enzyme. Pretrypsinization of the extract from the control cells also resulted in a new peak of activity that was eluted in fractions containing low molecular mass proteins. The above data indicate that removal of a carboxyl-terminal segment from the enzyme by trypsin treatment results in the formation of a water-soluble, low molecular mass, constitutively active enzyme.
When extracts of BHK cells expressing a truncated PTPase in which an 11-kDa segment was deleted from the carboxyl end (TCΔC11.PTPase-transfected cells) were fractionated as described above and assayed without prior trypsin treatment, only about 50% of the PTPase activity sedimented with the 5P fraction, while the remainder was in the 100S supernatant (Table 1). However, the enzyme present in the 5P pellet was fully active without trypsin treatment and could be extracted with either 0.5% Triton X-100 or 0.6 M KCl, indicating that it was not as tightly associated with the particulate fraction as was the full-length PTPase. Although the total phosphatase activity in the cells expressing the truncated enzyme was the same as that in the controls, it differed in that it was 8-fold higher in the 100S fraction. When BHK cells expressing the truncated PTPase were extracted with the Triton buffer and subjected to Superose 12 gel filtration, little of the activity distributed with the high molecular mass complex; indeed, it was detected only in low molecular mass fractions of approximately 35 kDa as confirmed by Western blot analysis. BHK cells transfected with this truncated form of the PTPase exhibited only about 50% of the growth rate of the control cells or cells transfected with the wild-type enzyme.
To determine the state of activity of the enzyme *in vivo,* cells expressing both forms of the T-cell PTPase were stimulated with PDGF, and changes in protein-tyrosine phosphorylation were investigated. The BHK cells were treated with 80 µM ZnSO₄, followed by a 48-hour incubation in medium and in 0.1% heat-inactivated Fetal Calf Serum for 48 hours, which renders the cells quiescent. Serum-deprived cells were treated with 40 ng of platelet-derived growth factor (PDGF) (Amgen Biologicals) per ml at 37°C for various times and then washed immediately with ice-cold PBS. Lysis buffer (1 ml) containing 50 mM Hepes (pH 7.5), 150 mM NaCl, 10% (vol/vol) glycerol, 1% Triton X-100, 1.5 mM MgCl₂, 1 mM EGTA, 10 µg of aprotinin per ml, 2 µg of leupeptin per ml, 0.002% phenylmethylsulfonyl chloride, 200 µM sodium orthovanadate, 10 mM sodium pyrophosphate, and 100 mM NaF was added to the plates, which were then incubated on ice for approximately 20 min. The lysates were centrifuged at 10,000 x g for 5 min. at 4°C, and the protein concentrations were determined as described by Bradford (Anal. Biochem. 72:248-254, 1976). Thirty microliters of a suspension of agarose-linked mouse monoclonal anti-phosphotyrosine antibody beads (Oncogene Science, Manhasset, N.Y.) was added to equivalent amounts of lysate protein in each immunoprecipitation, and the mixture was rotated overnight at 4°C. The beads were collected by centrifugation, washed twice in 20 mM Hepes, pH 7.5/150 mM NaCl/0.1% Triton X-100/10% glycerol/200 µM orthovanadate, then twice again with the same buffer but with increasing salt concentration to 0.5 M NaCl, and finally with buffer containing 150 mM NaCl. The beads were boiled for 2 min. in 30 µl of Laemmli sample buffer (Laemmli, Nature 227:680-685, 1970). The immunoprecipitated protein was subjected to Western blot analysis (Ausubel, F.M. et al., eds., Current Protocols in Molecular Biology, Wiley, N.Y., Vol. 2, 1988) with an anti-phosphotyrosine antibody. To detect antibody binding, ¹²⁵I-labeled protein A (New England Nuclear, Boston, Mass.; 500,000 cpm/ml in 10 mM Tris, pH 7.4/150 mM NaCl/1% bovine serum albumin) was added to the blot for 2 hr. and washed in 10 mM Tris, pH 7.4/150 mM NaCl/0.05% Triton X-100. The blot was then subjected to autoradiography for 2-5 days at room temperature.
As described in detail immediately above, serum-deprived BHK cells were stimulated with PDGF and extracted at various times in the presence of vanadate, and proteins phosphorylated on tyrosyl residues were immunoprecipitated with anti-phosphotyrosine antibody. The precipitated proteins were subjected to SDS/PAGE and then analyzed in a Western blot with a second anti-phosphotyrosine antibody. Autoradiography of the blot (Figure 2) revealed that 2 min. after PDGF stimulation, there was a dramatic increase in tyrosine phosphorylation in proteins of approximately 180, 140, 116, 92, and 60 kDa in the control cells. On the other hand, in cells overexpressing either the wild-type or truncated T-cell PTPase, there was a considerably lower level of phosphorylation in several protein bands, particularly those of 140, 116, and 60 kDa but not that of the 180-kDa protein assumed to be the PDGF receptor. The 116- and 60-kDa proteins appeared to undergo dephosphorylation in extracts from control cells within this time period, suggesting that an endogenous PTPase was activated. Similar phosphoproteins were detected in phosphotyrosine immunoprecipitates from quiescent fibroblasts treated with PDGF or normal fibroblasts pretreated with vanadate.
In summary, the results show that the overexposed full-length 48-kDa T cell PTPase localizes to a particulate fraction that sediments at low speed. The enzyme is inactive *in vitro* toward RCM-lysozyme unless the fraction is pretreated with trypsin. This behavior can be ascribed to the 11-kDa carboxyl-terminal segment of the molecule. The enzyme is active *in vivo.* Despite this activity, there was no effect on BHK growth rate or gross morphology (by phase-contrast microscopy; however, there was an effect on the cytoskeleton) as compared with cells expressing the vector alone. It appears that the enzyme is highly regulated within the cells and/or it may be partitioned into a compartment(s) where its activity would be restricted to specific substrates. The results also show that the overexpressed 37-kDa truncated form exhibits behavior different from the full-length enzyme. This truncated form is constitutively active and distributes evenly between particulate and soluble fractions during purification. The BHK cells in which it was expressed grew at a reduced rate and displayed gross morphological changes. Therefore, it appears that the carboxyl-terminal segment is involved in the localization and regulation of enzyme activity.
A hydrophobicity plot (Figure 3) indicates that this segment is essentially hydrophilic until approximately the last 20 residues, at which point the polypeptide chain becomes hydrophobic, with a hydrophobicity index approaching that of transmembrane segments or signal peptides. A similar distribution of hydrophobic and hydrophobic residues is also found in the carboxyl-terminal segments of low molecular mass human placenta and rat brain PTPases, even though the primary structure of these segments is more variable than within their conserved 236-residue core structure. Sucrose density centrifugation gave no evidence for association of the T-cell PTPase enzyme with the plasma membrane. However, this PTPase might be interacting with other cellular components (nucleus, Golgi, or endoplasmic reticulum) or the cytoskeleton, since both high salt concentration and detergents are required for solubilization.
B. Baculovirus System
Wild type T cell cDNA and truncated T cell cDNA were introduced into Sf 9 (Spodoptera frugiperda) insect cells according to the method of Summers and Smith, Texas Agriculture Experimental Station Bulletin No. 1555, 1987. Briefly, the cDNA encoding T cell PTPase and truncated T cell PTPase was inserted into the BamHI site that is 3' to the polyhedron promoter in the Baculoviral expression plasmid. The recombinant baculoviral DNA was cotransfected with the normal baculoviral genome into host Sf 9 insect cells (see Summers et al., *supra*). DNA was taken up by the cells, the recombinant plasmid and baculoviral DNA underwent genetic recombination *in vivo* and recombinant virus were generated. These viruses were selected by hybridization of the ³²P-labeled T cell PTPase cDNA to insect cell lysates. The mRNA encoding PTPase protein was transcribed from the viral polyhedron promoter and the protein synthesized by the infected Sf 9 host cells. Approximately 30% of the total protein produced by the insect Sf 9 cells was due to the production of PTPase.
More specifically, open reading frames of the full-length 48-kDa T cell PTPase (TC.PTPase) and the 37-kDa truncated T cell PTPase (TCΔC11.PTPase) were isolated by a ThaI/SspI digest (Examples 1 and 2, above) and cloned into the unique BamHI site of the plasmid pVL 941 (Luckow and Summers, Virology 170:31-39, 1989) after filling in the sticky ends with Klenow fragment and deoxynucleotides (Pharmacia). Correct orientation of insertion was confirmed by DNA sequence analysis (Sanger et al., Proc. Natl. Acad. Sci. USA 74:5463-5467, 1977).
Sf9 cells were maintained in monolayer cultures as described (Summers and Smith, 1987). Cells were grown in Grace's Antheraea medium (Grace, Nature 195:788-789, 1962; Gibco, Grand Island, N.Y.) supplemented with 3.3 g/L yeastolate (Difco Labs, Detroit, MI), 3.3 g/L lactalbumin hydrolysate (Difco Labs, Detroit, MI), 10% fetal calf serum (Hink, Nature 226:446-467, 1970), and 100 U/mL penicillin, 100 µg/mL streptomycin and 0.25 µg/mL fungizone (Fungibact antibiotic mix, Irving Scientific, Santa Ana, Calif.). Cells were cotransfected with 1 µg Ac-NPV DNA and 2 µg plasmid (pAc-Tc.PTPase) as described by Summers and Smith, 1987. Purification of recombinant viruses was achieved by five rounds of serial end-point dilution and dot hybridization using ³²P-labelled TC.PTPase cDNA as a probe. Purity of the final virus suspension was checked by hybridization with a ³²P-labelled oligonucleotide probe; its sequence consists of nucleotides 37 to 66 of the Ac-NPV polyhedrin gene (Iddekinge et al., Virology 131:561-565, 1983), a portion missing in the pVL 941 expression vector. Extracts of Sf9 cells infected with pure recombinant virus do not hybridize with this oligonucleotide.
Cells were infected at a high (>3) multiplicity of infection and grown at 27°C. At different times post infection, cells were harvested by centrifugation for 5 min. at 5,000 x g. The following buffers were routinely used for extraction: (a) low salt buffer consisting of 25 mM imidazole pH 7.2, 2 mM EDTA, 0.1% 2-mercaptoethanol, 1 mM benzamidine, 0.002% phenylmethylsulfonyl fluoride, 2 µg/mL leupeptine, 1 µg/mL pepstatin, 5 kallikrein U/ml aprotinin; (b) low salt buffer containing 0.5% Triton X-100; (c) high salt buffer containing 0.6 M KCl and 0.5% Triton X-100 or 1% CHAPS.
Cells were suspended in low salt buffer (3 x 10⁷ cells/ml) and disrupted by 30 strokes in a Dounce homogenizer on ice. After 10 min. centrifugation at 10,000 x g, the pellet was resuspended in half of the original volume of low salt buffer containing 0.5% Triton X-100. The suspension was homogenized as previously described and centrifuged for 10 min. at 100,000 x g. Finally, the pellet was resuspended in high salt buffer containing 0.5% Triton X-100 or 1% CHAPS. The homogenate was again centrifuged for 10 min. at 100,000 x g; the supernatants were used for Western blot analysis, protein purification and PTPase assays. For Western blot analysis, proteins were subjected to SDS-PAGE as described by Laemmli (Nature 227:680-685, 1970) and electrophoretically transferred to nitrocellulose. Rabbit antibody 8172 raised against a synthetic peptide derived from the amino terminal region of PTPase 1B (see Example 4 above) was used for detection in Western blots. Goat anti-rabbit IgG conjugated to alkaline phosphatase (Bio-Rad Laboratories, Richmond, Calif.) was used as a secondary antibody according to the manufacturer's instructions.
For purification of TC.PTPase, the Triton/KCl extract was made 20% in ammonium sulfate and centrifuged for 10 min. at 10,000 x g. The precipitate was resuspended in 200 µL high salt buffer containing 0.5% Triton X-100 and applied to a Superose 12 FPLC column equilibrated in the same buffer. For purification of the C-terminal truncated enzyme, the low salt buffer extract was directly applied to a Sephadex G75 superfine column (2.6 cm x 82.5 cm, flow rate ca. 10 mL/h). Peak fractions were made 20% in glycerol and frozen at -70°C.
PTPase activity was measured as described in Example 3 above, except in some assays phosphorylated myelin basic protein (MBP) was used in saturating concentrations as substrate instead of tyrosyl phosphorylated RCM-lysozyme (RCML). Trypsin treatment was carried out by incubating the PTPase (<0.25 U/ml) in 40 µl of assay buffer with 1 µg of trypsin for 5 min. at 30°C. Trypsin was inhibited by addition of 6 µg of lima bean trypsin inhibitor, and the phosphatase reaction was started by addition of substrate.
The internal kinase domain of the epidermal growth factor receptor (EGFR) as expressed in the baculovirus system (Hsu et al., Cell Growth Differ. 1:191-200, 1990) was autophosphorylated in 20 mM HEPES pH 7.5, 0.1% 2-mercaptoethanol, 5% glycerol, 1 µg/mL pepstatin, 5 kallikrein U/mL aprotinin, 2 µg/mL leupeptin, 5 mM manganese acetate and 0.1 mM ATP (2.2 x 10⁵ cpm/pmol) for 10 min. at 30°C. The kinase reaction was stopped by adding EDTA to a concentration of 10 mM. The autophosphorylated receptor (80 ng) was then incubated either with buffer or with 100 ng of TC.PTPase or TCΔC11.PTPase, respectively, for 20 min. at 30°C. Sample buffer was added and the reaction run onto a 7.5% SDS-PAGE. The gel was dried and autoradiographed.
As described above, Sf9 cells were cotransfected with pAc-TC.PTPase plasmid DNA and Ac-NPV wild type DNA. At different times post infection, cells were harvested and lysed; the lysates were subjected to SDS-PAGE and immunoblot analysis. Uninfected cells or cells infected with wild type (wt) Ac-NPV served as controls. The expression level (observed by Western blot analysis) of both TC.PTPase and TCΔC11.PTPase increased steadily from day 2 to 5; after 5 days, the majority of cells had lysed due to the viral infection. Additional bands of higher Mr appeared after 3 days, probably due to some post-translational modification. Limited trypsinolysis of both proteins gave rise to a tryptic fragment of approximately 33 kDa. Since antibody 8172 recognizes a sequence near the N-terminus C-terminal of the enzyme, the main tryptic cleavage must have occurred in the region of the protein. No cross-reacting material was detected in uninfected cells or in cells infected with the wt virus. The increase PTPase activity in extracts from infected Sf9 cells paralleled the increased level of expression as observed by Western blot analysis. Substantial activity of the TC.PTPase towards RCML could be seen only following limited trypsinolysis of the enzyme.
The cells were extracted first in low salt buffer and the suspension centrifuged. The pellet was re-extracted with buffer containing 0.5% Triton X-100; this suspension was recentrifuged, and the second pellet was extracted with the same Triton buffer but in the presence of 0.6 M KCl. The full-length enzyme could be solubilized only by such a combination of salt and detergent; in contrast, the truncated form was readily soluble in aqueous buffer.
The TC.PTPase from Triton/KCl extracts was precipitated by adding ammonium sulfate to 20% saturation. The suspension was centrifuged and the pellet solubilized in Triton/KCl buffer. Although the recovery was only ca. 50%, this step was necessary to concentrate the enzyme. The protein solution was applied to a Superose 12 FPLC column. TC.PTPase eluted as one major peak of activity with a trailing shoulder. When fractions from both (18-20 and 21-24) were collected, the material in each pool resulted in the same band pattern on SDS-PAGE displaying a doublet at 48 kDa and a faint band at 40 kDa. Due to contaminants, the specific activity of the material in the trailing edge was approximately half that of the forward peak. Both fractions eluted at molecular weights (220 k and 160 k, respectively) higher than expected for the monomeric molecule (48 k), implying aggregation, insertion into detergent micelles or asymmetry of the molecule.
Aggregation of the protein could not be demonstrated directly. Assuming that the activation of the enzyme by polyamines or MBP resulted from disaggregation, the full-length PTPase was chromatographed on the same FPLC Superose 12 column after equilibrating the column in 2 mM spermine or after preincubation with a ten-fold molar excess of unphosphorylated MBP. No shift in the elution pattern was observed. Furthermore, attempts at cross-linking with dimethylsuberimidate revealed no protein species with a Mr higher than 4 k on SDS-PAGE, whereas cross-linking of hemoglobin as a control under the same conditions was successful.
To test the possible influence of the detergent, chromatography was also carried out in CHAPS which forms smaller micelles (ca. 5 kDa than Triton X-100 (ca. 90 kDa). However, here again, the enzyme eluted at a Mr higher than expected (ca. 170 k).
All three band visible in SDS-PAGE correspond to different forms of the TC.PTPase since they are recognized by antibody 8172 and two other antibodies directed against different segments of the T-cell PTPase (residues 342-357 and 369-381). Further characterization of the enzyme was performed on material obtained from peak fractions 19 and 20.
Since 90% of TCΔC11.PTPase distributed in the aqueous buffer, the extract could be applied directly to a Sephadex G75 superfine column. In contrast to the full-length protein, TCΔC11.PTPase eluted at its expected molecular weight. Table 2 summarizes the purification of both forms of the T-cell PTPase. These could be stored for months in the presence of 20% glycerol at -70°C without significant loss of activity.

**TABLE 2**

| Purification of expressed TC.PTPase (A) and TCΔC11.PTPase (B) | | | | | | |
|---|---|---|---|---|---|---|
| **A** | volume | total activity^{a} | protein | specific activity^{a} | purification | yield |
| | [ml] | [units] | [mg] | [units/mg] | [fold] | [%] |
| Triton/KCl extract | 1.0 | 12,000 | 4.6 | 2.650 | 1 | 100 |
| (NH₄)₂SO₄ precipitate | 0.2 | 6,400 | 2.3 | 2,850 | 1 | 53 |
| Superose 12 peak | 0.5 | 3,250 | 0.3 | 10,700 | 4 | 27 |

| **B** | volume | total activity^{b} | protein | specific activity^{b} | purification | yield |
|---|---|---|---|---|---|---|
| | [ml] | [units] | [mg] | [units/mg] | [fold] | [%] |
| extract | 3.2 | 14,900 | 4.65 | 3,200 | 1 | 100 |
| G75 Sephadex peak | 9.0 | 7,200 | 0.24 | 30,200 | 9 | 48 |

| | | | | | | |
|---|---|---|---|---|---|---|
| ^{a} activities determined with MBP as substrate in the presence of 5 mM EDTA | | | | | | |
| ^{b} activities determined with RCML as substrate in the presence of 5 mM EDTA | | | | | | |

Both forms of the T-cell PTPase were totally specific for phosphotyrosyl residues showing no activity towards MBP or histones phosphorylated by the cAMP-dependent protein kinase. The truncated form of the PTPase displayed a specific activity of 26,000 U/mg toward tyrosyl phosphorylated RCML. By contrast, the full-length enzyme was far less active (850 U/mg) suggesting that enzyme activity is repressed by the C-terminal segment (Table 3) Both limited trypsinolysis and truncation of the molecule by introduction of a premature stop codon led to a 30-fold increase in activity toward RCML. The activity of the full-length enzyme depended greatly on the nature of the substrate. In the presence of phosphorylated MBP, it displayed a specific activity of 10,300 U/mg as compared to 4,700 U/mg only for the truncated form. These data suggest that MBP interacts with the C-terminal segment, resulting in an activation of the enzyme. Both forms of the T-cell PTPase readily dephosphorylated the soluble kinase domain of the EGFR following its autophosphorylation.

**TABLE 3**

| Enzymatic Properties of PTPase Forms | | | | |
|---|---|---|---|---|
| Substrate: | RCML | | MBP | |
| | specific activity^{a} | Kₘ | specific activity^{a} | Km |
| | [units/mg] | [nM] | [units/mg] | [nM] |
| TC.PTPase | 850 ± 170 | 200 | 10,300 ± 1,300 | 500 |
| after trypsinolysis | 23,300 ± 3,800 | n.d. | 3,600 ± 1,000 | n.d. |
| | | | | |
| TCΔC11.PTPase | 26,000 ± 3,000 | 50 | 4,700 ± 500 | 1250 |
| after trysinolysis | 15,300 ± 2,600 | n.d. | 1,900 ± 200 | n.d. |

| | | | | |
|---|---|---|---|---|
| ^{a} average and standard deviation for three separate preparations measured under conditions of substrate saturation in the presence of 5 mM EDTA | | | | |

Both forms of the T cell enzyme were inhibited by micromolar concentrations of the classical inhibitors vanadate (Swarup et al., Biochem. Biophys. Res. Commun. 107:1104-1100, 1982), molybdate and Zn²⁺ (Brautigan et al., J. Biol. Chem. 256:6519-6522, 1981) (Table 4). Calcium and magnesium were essentially without effect. Both forms of the T cell enzyme were inhibited by nanomolar concentrations of polyanions; this effect was only observed with RCML as substrate. The enzyme was activated by EDTA, but not as potently as PTPase 1B. The full-length enzyme was markedly activated by polycationic compounds (up to sevenfold by spermine and up to threefold by unphosphorylated MBP) only when negatively charged RCML was used as the substrate. By contrast, activation of the truncated form of the enzyme by these compounds was 30% at most, suggesting that the polycationic molecules interact with the C-terminal region.

**TABLE 4**

| Effectors of PTPase Activity | | | | |
|---|---|---|---|---|
| Activity is expressed as the percentage of phosphate released relative to control in which the effector was omitted. All assays were performed in duplicate in the presence of 5 µM substrate. | | | | |

| Enzyme form: | TC.PTPase | | TCΔC11.PTPase | |
|---|---|---|---|---|
| Substrate: | RCML | MBP | RCML | MBP |
| effector | | | | |
| none | 100 | 100 | 100 | 100 |
| 100 µM vanadate | 0 | 0 | 2 | 1 |
| 10 µM molybdate | 0 | 1 | 2 | 2 |
| 100 µM Zn⁺⁺ | 33 | 101 | 15 | 66 |
| | | | | |
| 0.01 µM heparin | 96 | 120 | 58 | 102 |
| 1 µM heparin | 8 | 108 | 8 | 75 |
| 10 µM heparin | 5 | 73 | 1 | 90 |
| | | | | |
| 0.01 µM poly Glu:Tyr 4:1 | 66 | 119 | 70 | 102 |
| 1 µM poly Glu:Tyr 4:1 | 18 | 133 | 17 | 104 |
| 10 µM poly Glu:Tyr 4:1 | 6 | 58 | 11 | 127 |
| | | | | |
| 5 mM EDTA | 124 | 137 | 170 | 150 |
| | | | | |
| 2 mM spermine | 727 | 62 | 110 | 97 |
| 2 mM spermidine | 249 | 43 | 132 | 108 |
| | | | | |
| 1 µM unphosphor. MBP | 122 | 62 | 110 | 97 |
| 10 µM unphosphor. MBP | 297 | 43 | 132 | 108 |
| 50 µM unphosphor. MBP | 279 | 15 | 10 | 66 |

In addition to the method described above, U.S. Patent Nos. 4,879,236; 4,870,023; and 4,745,051 (herein incorporated by reference) disclose methods for producing recombinant baculoviruses and their infection of insect cells.
C. Retroviruses
Wild type T cell PTPase (48 kDa) cDNA or c-terminal truncated PTPase cDNA are introduced into cells according to the method of Miller and Rosman (BioTechniques 7:980-990, 1989). The cDNAs of interest are ligated into the vector LXSN (kindly provided by Dr.Miller, Fred Hutchinson Cancer Research Center) and transfected by the calcium phosphate precipitation method into a retroviral packaging cell line (PE501) (provided by Dr. Miller). The cells which contain the DNA are resistant to the drug G418 and thus are selected for their ability to grow in the presence of 1.5 mg/ml of the drug in the media Individual colonies are isolated and their supernatants containing recombinant retroviruses are saved.
A number of cell lines are appropriate to test whether the retroviruses are infectious and are producing the PTPases *in vivo*. These include NIH 3T3, NIH Swiss D1, NIH 3T3 transformed by v-src, Rat 2, and Rat 2 transformed by v-fms. Approximately 10⁵ cells are plated, infected with 100 µl of retroviral supernatant from each of the colonies of retroviral producing cell lines. Positive infection is tested by selection in G418 media. Finally, any G418-resistant cells are analyzed for expression of either the 48 kDa or the c-terminal truncated PTPase by immunoprecipitation with a specific anti - T cell PTPase antibody (e.g., the antisera of Example 4).

### EXAMPLE 6

### REVERSION OF MALIGNANT TRANSFORMATION OF BHK CELLS BY TRANSFECTION WITH T CELL CDNA

DNA encoding a modified form of the 48 kDa form of the T cell PTPase was introduced into Baby Hamster Kidney (BHK) cells. The modification of the cDNA is a deletion of an 11 kDa carboxy-terminal extension which renders the enzyme very active with a specific activity of 40,000 units/mg *in vitro*. The expression of this modified PTPase is not toxic to the BHK cells. In addition, BHK cells expressing this enzyme exhibited an altered phenotype in that they were no longer transformed.

The nature of the transformation of baby hamster kidney (BHK) cells is unknown. Two standard techniques for determining oncogenicity are: (1) to grow cells in soft agar demonstrating that the cells are no longer contact inhibited, and (2) to inject cells into nude mice (i.e., mice which are lacking a thymus and so cannot reject the cells) and look for tumor growth at the point of injection. These tests were positive for BHK cells, BHK cells transfected with an expression vector, BHK cells transfected with the 48 kDa form of the T cell PTPase, but negative for the cells expressing high levels of the c-terminal truncated form of the T cell PTPase.

For example, the control cells showed colonies in soft agar in two weeks which were much greater than 2 µm in diameter whereas all the colonies containing cells expressing the truncated PTPase were less than 2 µm. This was true for cells grown up to one month in soft agar. The volumes of the tumors formed after one month in nude mice which had been injected with the control cells, i.e., 2.5 x 10⁶ cells expressing either the vector or the 48 kDa enzyme, were 15 ml and 2.1 ml, respectively, and these large tumors undergo angiogenesis. The volumes in nude mice injected with 5 x 10⁶ cells expressing a c-terminal truncated form of the T cell PTPase ranged from 0.21-0.38 ml, and these small growths do not undergo angiogenesis.

## Claims

1. A gene transfer vehicle capable of infecting malignant cells, said gene transfer vehicle carrying a DNA construct comprising a DNA molecule encoding a receptor-linked protein tyrosine phosphatase.

2. The gene transfer vehicle according to Claim 1 wherein said DNA molecule encodes a receptor-linked protein tyrosine phosphatase having a truncated carboxyl terminus.

3. A gene transfer vehicle according to Claim 1 or 2 wherein said gene transfer vehicle comprises a recombinant retrovirus or a recombinant vaccinia virus.

4. A gene transfer vehicle according to any one of Claims 1 to 3 for use within a method for treating a malignancy in a warm-blooded animal, wherein a protein tyrosine kinase is associated with the malignancy.

## Patentansprüche

1. Ein Gentransfervehikel, das in der Lage ist, maligne Zellen zu infizieren, wobei besagtes Gentransfervehikel ein DNA-Konstrukt trägt, das ein DNA-Molekül umfaßt, das eine rezeptor-verknüpfte Proteintyrosinphosphatase codiert.

2. Das Gentransfervehikel nach Anspruch 1, wobei besagtes DNA-Molekül eine rezeptor-verknüpfte Proteintyrosinphosphatase mit einem verkürzten Carboxyl-Terminus codiert.

3. Ein Gentransfervehikel nach Anspruch 1 oder 2, wobei besagtes Gentransfervehikel ein rekombinantes Retrovirus oder ein rekombinantes Vacciniavirus umfaßt.

4. Ein Gentransfervehikel nach einem der Ansprüche 1 bis 3 zur Verwendung in einem Verfahren zur Behandlung einer bösartigen Erkrankung in einem Warmblüter, wobei eine Proteintyrosinkinase mit der bösartigen Erkrankung assoziiert ist.

## Revendications

1. Vecteur de transfert de gènes apte à l'infection de cellules malignes, ledit vecteur de transfert de gènes portant un produit d'assemblage d'ADN comprenant une molécule d'ADN codant pour une protéine-tyrosine-phosphatase liée à un récepteur.

2. Vecteur de transfert de gènes suivant la revendication 1, dans lequel la molécule d'ADN code pour une protéine-tyrosine-phosphatase, liée à un récepteur, ayant une extrémité carboxy-terminale tronquée.

3. Vecteur de transfert de gènes suivant la revendication 1 ou 2, qui comprend un rétrovirus recombinant ou un virus vaccinal recombinant.

4. Vecteur de transfert de gènes suivant l'une quelconque des revendications 1 à 3, destiné à être utilisé dans une méthode de traitement d'une affection maligne chez un animal à sang chaud, une protéine-tyrosine-kinase étant associée à l'affection maligne.
